# EUROPEAN PATENT APPLICATION

(11) **EP 3 698 741 A1**
(43) Date of publication of application: **26.08.2020**
(21) Application number: 20158723.5
(22) Date of filing: 21.02.2020
(51) Int. Cl.: A61B 17/34

(54) **ACCESS ASSEMBLY INCLUDING FLEXIBLE CANNULA**

(30) Priority: 22.02.2019 US 201916282375
(71) Applicant: Covidien LP, Mansfield, MA 02048 (US)
(72) Inventor: ROBINSON, William, E, Boulder, CO 80301 (US); ALLEN IV, James D., Broomfield, CO 80020 (US); HAMMERLAND III, John, A, Arvada, CO 80007 (US)
(74) Representative: Maschio, Antonio

(57) **Abstract**

An access device (100) configured for accommodating passage of an instrument (10) with a curved end effector (12) includes a housing assembly (110) and a cannula member (120) extending from the housing. The cannula member includes a length and a plurality of rigid portions alternating with a plurality of flexible portions. The cannula member is configured to maintain a circular cross-sectional profile when a force is applied to the cannula member from the outside and to flex to an oblong cross-sectional profile when a force is applied to the cannula member from the inside.

## Description

### BACKGROUND

### Technical Field

The present disclosure relates to an access assembly for use in laparoscopic surgical procedures. More particularly, the present disclosure relates to an access assembly including a flexible cannula for receiving curved instruments.

### Background of Related Art

Endoscopic surgical procedures are known. The term endoscopic as used herein is defined to include all types of minimally invasive surgical procedures including laparoscopic and arthroscopic procedures. Typically, during these procedures, after an incision has been formed in the body tissue, a cannula defining a lumen is inserted through the incision and positioned in relation to the surgical site.

To facilitate insertion of the cannula through the incision, and to maintain the lumen in an open position, the cannulas are generally rigid. In order to insert an endoscopic instrument having curved end effector through a rigid cannula, a larger diameter cannula is required. Larger cannulas necessitate a larger incision, which translates to a larger scar and increased healing time.

To accommodate passage of endoscopic instruments have a curved end effector, it would be beneficial to have a flexible cannula.

### SUMMARY

An access device configured for accommodating passage of an instrument with a curved end effector is provided. The access device includes a housing assembly, and a cannula member extending from the housing. The cannula member includes a length and a plurality of rigid portions alternating with a plurality of flexible portions. The cannula member is configured to maintain a circular cross-sectional profile when a force is applied to the cannula member from the outside and to flex to an oblong cross-sectional profile when a force is applied to the cannula member from the inside.

In embodiments, the circular cross-sectional profile defines a first diameter and the oblong cross-sectional profile defines a major axis. The major axis may be longer than the first diameter. Each rigid portion of the plurality of rigid portions may include a substantially "Z" shaped cross-sectional profile. Each rigid portion of the plurality of rigid portions may include a circular cross-sectional profile. Each rigid portion of the plurality of rigid portions may include an elongate arcuate cross-sectional profile.

The cannula member defines a thickness. Each of the plurality of rigid portions may extend the thickness of the cannula member. The cannula member may include an inner layer. The cannula member may include an outer layer. The inner and outer layers may form sleeve about the plurality of rigid and flexible portions. The plurality of flexible portions may integrally formed. The plurality of flexible portions may form channels through which the plurality of rigid portions is received.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:
FIG. 1 is a perspective view of an access assembly including a flexible cannula according to an embodiment of the present disclosure;
FIG. 2 is an end view of the flexible cannula shown in FIG. 1 in a first or natural condition;
FIG. 3 is an view end view of the flexible cannula shown in FIG. 1 in a second or oblong condition;
FIG. 4 is a cross-sectional side view of the access assembly shown in FIG. 1 with a curved end effector received in a housing assembly of the access assembly;
FIG. 5 is a cross-sectional side view of the access assembly shown in FIG. 4 with the curved end effector partially received through the flexible cannula;
FIG. 6 is a cross-sectional side view of the access assembly shown in FIGS. 4 and 5 with the end effector fully received through the access assembly;
FIG. 7 is a cross-sectional end view of a flexible cannula assembly according to an embodiment of the present disclosure;
FIG. 8 is a cross-sectional end view of a flexible cannula assembly according to another embodiment of the present disclosure;
FIG. 9 is a cross-sectional end view of a flexible cannula assembly according to yet another embodiment of the present disclosure; and
FIG. 10 is a cross-sectional end view of a flexible cannula assembly according to still another embodiment of the present disclosure.

### DETAILED DESCRIPTION

The access assemblies herein disclosed may be configured for use in various surgical procedures, including laparoscopic, endoscopic, arthroscopic and orthopedic surgery. The access assemblies provide passage between the outside atmosphere and a subject's body cavity. The access assemblies are capable of receiving surgical instruments of various sizes and configurations. Embodiments of the presently disclosed access assemblies are configured to receive, for example, clip appliers, graspers, dissectors, retractors, staplers, laser probes, photographic devices, endoscopes and laparoscopes, tubes, and the like, and more particular, instruments having a curved end effector. Such devices are collectively referred to herein as "instruments" or "instrumentation."

Referring now to the drawings wherein like reference numerals illustrate similar components throughout the several views, there are illustrated embodiments of access assemblies according the principles of the present disclosure. As shown in the drawings and as described throughout the following description, as is traditional when referring to relative positioning on an object, the term "proximal" refers to the end of the apparatus which is closer to the user and the term "distal" refers to the end of the apparatus which is farther from the user.

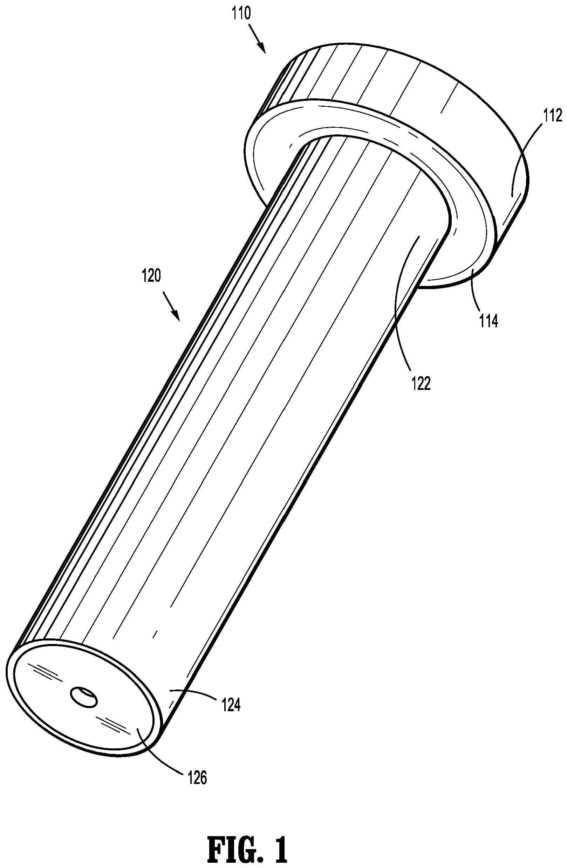

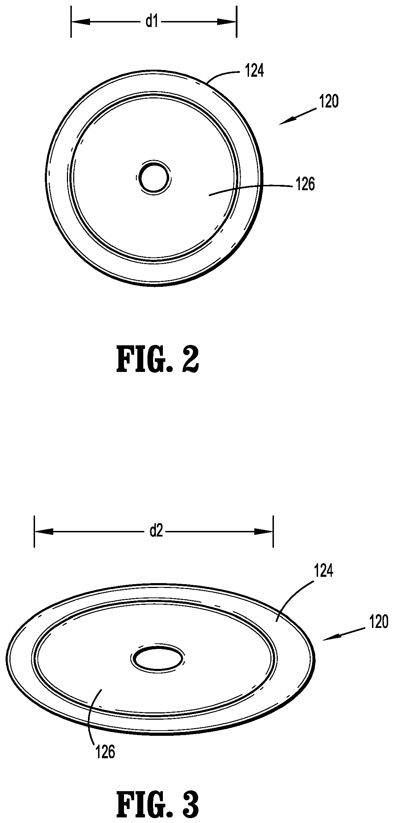

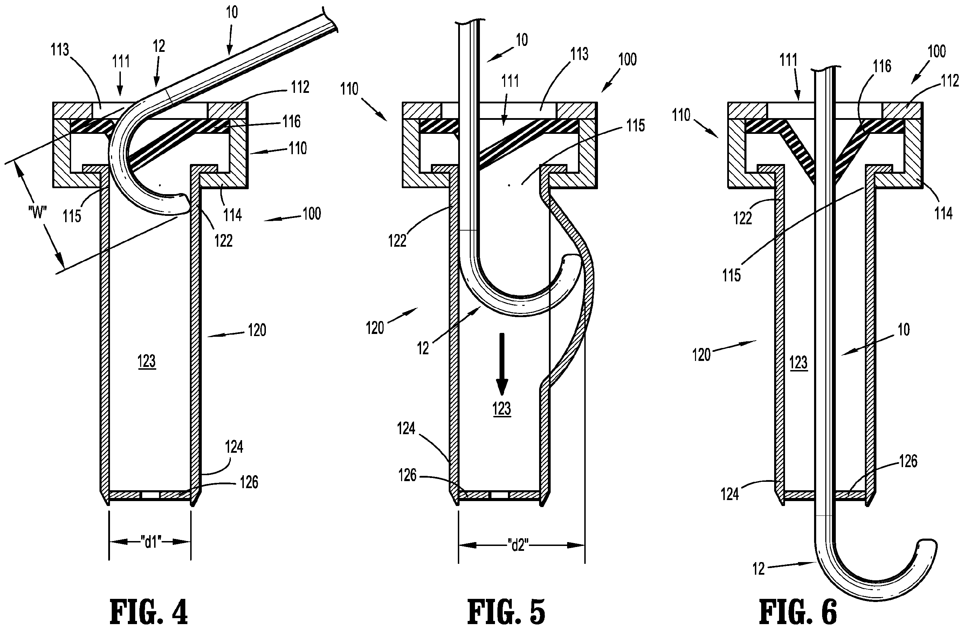

Referring initially to FIGS. 1 and 2, an embodiment of an access assembly according to the present disclosure is shown generally as access assembly 100. The access assembly 100 includes a housing assembly 110, and a cannula member 120 extending distally from the housing assembly 110. As will be discussed in further detail below, the access assembly 100 is configured to receive an instrument 10 (FIG. 4) having a curved end effector 12 with an effective width "W" (FIG. 4) greater than an inner diameter "d1" (FIG. 2) of the cannula member 120 when the cannula member 120 is in a first or natural state. In embodiments, the housing assembly 110 and the cannula member 120 are integrally formed, i.e., monolithic or unitary structure.

With reference still to FIGS. 1 and 2, the housing assembly 110 of the access assembly 100 includes a substantially cylindrical member having a proximal portion 112 defining an opening 113 (FIG. 4), a distal portion 114 defining an opening 115 (FIG. 4), and defining a passageway 111 (FIG. 5) therebetween. The distal portion 114 of the housing assembly 110 may be integrally formed with the cannula member 120 of the access assembly 100. Alternatively, the housing assembly 110 may be secured to the cannula member 120 with adhesive, welding, friction fit, mechanical fasteners, or with any other suitable method. In embodiments, the housing assembly 110 is configured for releasable engagement with the cannula member 120 to permit replacement of the cannula member 120.

The housing assembly 110 of the access assembly 100 may be constructed of plastic, polymer or other like material. In embodiments, the housing assembly 110 includes a low profile to accommodate receipt of the curved end effector 12 (FIG. 4) of the instrument 10 through the housing assembly 110. Alternatively, the housing assembly 110 may be formed of a flexible material to permit flexing of the housing assembly 110 to accommodate passage of the curved end effector 12 therethrough. In embodiments, the proximal portion 112 of the housing assembly 110 may be substantially open to accommodate receipt of a curved end effector through the housing assembly 110.

The housing assembly 110 of the access assembly 100 may be disposable, or may instead be configured for sterilization and reuse. The housing assembly 110 may include one or more seal members 116 (FIG. 4) having any seal arrangement suitable for receiving an instrument therethrough in a sealed manner. In embodiments, the housing assembly 110 includes a port (not shown) configured for connection to a source of insufflation gas.

Still referring to FIGS. 1 and 2, the cannula member 120 of the access assembly 100 forms a substantially tubular member having a proximal end 122 and a distal end 124, and defining a longitudinal passage 123 (FIG. 2) extending between the proximal and distal ends 122, 124. The cannula member 120 of the access assembly 100 is configured to be inserted into a body cavity through an incision in a patient. More particularly, the cannula member 120 may be configured to receive an obturator (not shown) having a blade or piercing tip for creating an incision to facilitate insertion of the cannula member 120 into a body cavity. Alternatively, the cannula member 120 may have a tapered or sharpened distal end 124 configured to be inserted through an incision.

The cannula member 120 of the access assembly 100 may include one or more seal members 126 (FIG. 4) disposed along its length. As will be described in detail below, the cannula member 120 is composed of a combination of rigid and flexible portions extending longitudinally along a length thereof. The arrangement of the rigid and flexible portions is such that the cannula member 120 is able to maintain a substantially circular cross-sectional profile (FIG. 2) when in a first or natural, unstressed configuration, and is able to deform to a second or flexed configuration defining an oblong cross-sectional profile (FIG. 3) capable of receiving an instrument having a curved end effector with an effective width greater than the inner diameter "d1" of the cannula in the natural configuration. Although the oblong cross-sectional profile is shown as being symmetric or oval, it is understood that the oblong cross-sectional profile may be asymmetric or ovate.

With reference to FIG. 2, in the first or natural configuration, the cannula member 120 of the access assembly 100 includes a substantially circular cross-sectional profile having an inner diameter "d1". The configuration of the cannula member 120 is such that the circular cross-sectional profile is maintained when forces act on the cannula member 120 from the outside, e.g., tissue through which the cannula is inserted. In this manner, the cannula member 120 does not collapse during insertion through an incision and/or while being maintained through an incision. As will be described in further detail below, the circular cross-sectional profile of the cannula member 120 is maintained through a combination of rigid and flexible portions extending the length of the cannula member 120.

Turning to FIGS. 3 and 4, when the instrument 10 having the curved end effector 12 with an effective width "W" greater than the inner diameter "d1" of the cannula member 120 in the natural configuration is inserted through the cannula member 120, the cannula member 120 is configured to deform into the oblong cross-sectional profile to accommodate the effective width "W" (FIG. 4) of the curved end effector 12. As shown in FIG. 3, the cannula member 120 is capable of accommodating an instrument having an effective width of "W" which is greater than the inner diameter "d1".

With reference to FIGS. 4-6, the curved end effector 12 of the instrument 10 is shown as the curved end effector 12 inserted through the access assembly 100. Referring initially to FIG. 4, the curved end effector 12 is first received through the housing assembly 110 of the access assembly 100 by inserting the curved end effector 12 through the opening 113 in the proximal portion 112 of the housing assembly 110. The curved end effector 12 is then passed through the seal member 116 before being manipulated through the opening 115 in the distal portion 114 of the housing assembly 110 into the cannula member 120.

As shown, the openings 113, 115 in the proximal and distal portions 112, 114, respectively, of the housing assembly 110 are smaller than the width "W" of the curved end effector 12. In this manner, a clinician must manipulate the instrument 10 to receive the curved end effector 12 through the housing assembly 110 and into the cannula member 120. In embodiments, the openings 113, 115 in the proximal and distal portions 112, 114, respectively housing assembly 110 may be of sufficient size to accommodate receipt of the curved end effector 12 therethrough without excessive manipulation of the instrument 10. Alternatively, and as noted above, the housing assembly 110 may be constructed of a material that permits flexing of the housing assembly 110 to accommodate the increased width of the curved end effector 12 during insertion of the curved end effector 12 through the housing assembly 110.

Turning to FIG. 5, after receiving the curved end effector 12 of the instrument 10 through the housing assembly 110 of the access assembly 100, the curved end effector 12 engages an inner surface of the cannula member 120. As shown in FIG. 5, the force of the curved end effector 12 against the inner surface of the cannula member 120 deforms the cannula member 120 from its initial configuration with the first inner diameter "d1" to the oblong configuration with the inner major axis "d2" to accommodate the effective width "W" of the end effector 12. As the curved end effector 12 is passed through the cannula member 120, the cannula member 120 deforms locally along its length to the oblong configuration to accommodate passage of the curved end effector 12.

As seen in FIG. 6, the cannula member 120 returns to its initial, circular configuration after the curved end effector 12 passes through the cannula member 120. The access assembly 100 then operates in a manner similar to traditional access assemblies.

The curved end effector 12 of the instrument 10 is removed through the access assembly 100 in the reverse order of its insertion. Alternatively, the access assembly 100 and the instrument 10 may be removed from the patient simultaneously, i.e., while the instrument 10 is still received through the access assembly 100, thereby eliminating the need for the curved end effector 12 to pass back through the cannula member 120 and the housing assembly 110.

Following the surgical procedure, the access assembly 100 may disposed of in a traditional manner, or be cleansed and sterilized for reuse. As noted above, either or both of the housing assembly 110 and the cannula member 120 may be disposable and/or configured for reuse.

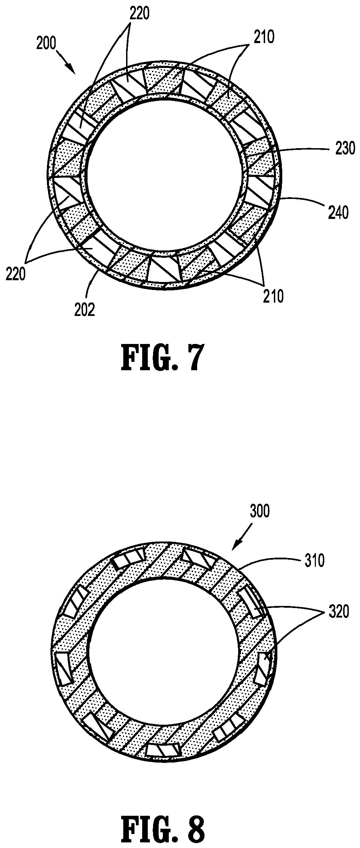

With reference now to FIGS. 7-10, embodiments of various cannulas configured to accommodate curved end effectors are shown.

Referring initially to FIG. 7, a cannula member according to an embodiment of the present disclosure is shown generally as cannula member 200. The cannula member 200 includes alternating flexible portions 210 and rigid portions 220 that extend the length of the cannula member 200. In embodiments, the rigid portions 220 extend substantially the depth of the cannula wall 202. The flexible portions 210 of the cannula member 200 are configured to permit the cannula member 200 to flex along its length during receipt of the curved end effector 12 therethrough. The rigid portions 220 of the cannula member 200 are configured to maintain the circular shape of the cannula member 200 prior to receipt of the curved end effector therethrough. In this manner, the flexible and rigid portions 210, 220 operate together to maintain the cannula member 200 in the initial configuration while stressed from the outside, and permit the cannula member 200 to flex outwardly to the oblong configuration while stressed from the inside.

The flexible portions 210 of the cannula member 200 may be formed of rubber, polymer, or other suitable material that permit flexing and/or stretching of the cannula member 200. The rigid portions 220 of the cannula member may be formed of plastic, metal, alloys or other rigid or semi-rigid material that maintain the structure of the cannula member 200. The flexible portions 210 and the rigid portions 220 may be secured to one another using adhesive, welding, or in any other suitable manner. In embodiments, the flexible portions 210 are over-molded about the rigid portions 220.

In embodiments, the cannula member 200 includes an inner layer 230 and/or an outer layer 240. The inner and outer layers 230, 240 may form a sleeve for maintaining the flexible portions 210 and rigid portions 220 of the cannula member 200 in alignment with each other. The inner and outer layers 230, 240 may be formed of rubber or other elastic material that permit flexing of the cannula member 200. In embodiments, the inner and outer layers 230, 240 are integrally formed with the flexible portions 210 and define longitudinal channels through which the rigid portions 220 are received. Alternatively, the inner and outer layers 230, 240 and flexible portions 210 of the cannula member 200 may be overmolded about the rigid portions 220. Although shown as having a substantially trapezoidal cross-sectional profile, the rigid portions 220 may include cross-sectional profiles of other shapes, including circular, oval, rectangular. The number and/or spacing of the flexible and rigid portions 210, 220 may be varied to yield particular expansion profiles.

Turning to FIG. 8, a cannula member according to another embodiment of the present disclosure is shown generally as cannula member 300. The cannula member 300 is substantially similar to cannula member 200 described hereinabove, and will only be described as to the differences therebetween. The cannula member 300 includes a flexible base portion 310 and rigid portions 320 reinforcing the flexible base portion 310. The rigid portions 320 may be received within preformed channels of the flexible base portion 310. Alternatively, the flexible base portion 310 may be over-molded about the rigid portions 320. The number, thickness, and/or spacing of the flexible and rigid portions 310, 320 may be varied to yield particular expansion profiles.

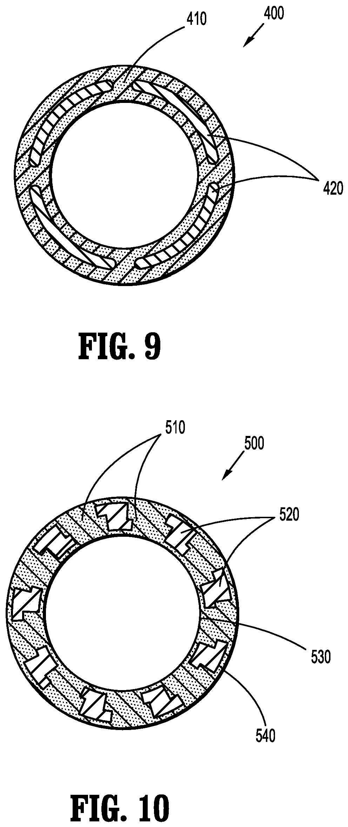

With reference now to FIG. 9, a cannula member according to yet another embodiment of the present disclosure is shown generally as cannula member 400. The cannula member 400 is substantially similar to the cannula members 200, 300 described hereinabove, and will only be described as relates to the differences therebetween. The cannula member 400 includes a flexible base portion 410 and rigid portions 420. The rigid portions 420 have an elongate arcuate cross-sectional profile. The rigid portions 420 may be received within preformed channels of the flexible base portion 410. Alternatively, the flexible base portion 410 may be over-molded about the rigid portions. The number, thickness, and/or spacing of the flexible and rigid portions 410, 420 may be varied to yield particular expansion profiles.

Referring now to FIG. 10, a cannula member according to still another embodiment of the present disclosure is shown generally as cannula member 500. The cannula member 500 is substantially similar to the cannula members 200, 300, 400 described hereinabove, and will only be described as relates to the differences therebetween. The cannula member 500 includes alternating flexible portions 510 and rigid portions 520. The rigid portions 520 include a substantially "Z" shaped cross-sectional profile. Accordingly, the flexible portions 510 include a corresponding substantially "Z" shaped cross-sectional profile. The cannula member 500 may include inner and outer flexible layers 530, 540 for maintaining the flexible portions 510 and the rigid portions 520 relative to each other. Alternatively, the flexible portions 510 and the rigid portions 520 may be secured to one another using know techniques. In embodiments, the flexible portions 510 and inner and outer layers 530, 540 are overmolded about the rigid portions 520. The number, thickness, and/or spacing of the flexible and rigid portions 510, 520 may be varied to yield particular expansion profiles.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described.

The invention may be described by reference to the following numbered paragraphs: -
1. An access device comprising: a housing assembly; and a cannula member extending from the housing and including a length, the cannula member including a plurality of rigid portions alternating with a plurality of flexible portions, the cannula member configured to maintain a circular cross-sectional profile when a force is applied to the cannula member from the outside and to flex to an oblong cross-sectional profile when a force is applied to the cannula member from the inside.
2. The access device of paragraph 1, wherein the circular cross-sectional profile defines a first diameter and the oblong cross-sectional profile defines a major axis, the major axis being longer than the first diameter.
3. The access device of paragraph 1, wherein each rigid portion of the plurality of rigid portions includes a substantially "Z" shaped cross-sectional profile.
4. The access device of paragraph 1, wherein each rigid portion of the plurality of rigid portions includes a circular cross-sectional profile.
5. The access device of paragraph 1, wherein each rigid portion of the plurality of rigid portions includes an elongate arcuate cross-sectional profile.
6. The access device of paragraph 5, wherein the cannula member defines a thickness, wherein each of the plurality of rigid portions extends the thickness of the cannula member.
7. The access device of paragraph 1, wherein the cannula member includes an inner layer.
8. The access device of paragraph 7, wherein the cannula member includes an outer layer.
9. The access device of paragraph 8, wherein the inner and outer layers form sleeve about the plurality of rigid and flexible portions.
10. The access device of paragraph 1, wherein the plurality of flexible portions are integrally formed.
11. The access device of paragraph 1, wherein the plurality of flexible portions form channels through which the plurality of rigid portions is received.

## Claims

1. An access device comprising:
a housing assembly; and
a cannula member extending from the housing and including a length, the cannula member including a plurality of rigid portions alternating with a plurality of flexible portions, the cannula member configured to maintain a circular cross-sectional profile when a force is applied to the cannula member from the outside and to flex to an oblong cross-sectional profile when a force is applied to the cannula member from the inside.

2. The access device of claim 1, wherein the circular cross-sectional profile defines a first diameter and the oblong cross-sectional profile defines a major axis, the major axis being longer than the first diameter.

3. The access device of claim 1, wherein each rigid portion of the plurality of rigid portions includes a substantially "Z" shaped cross-sectional profile.

4. The access device of claim 1 or claim 2, wherein each rigid portion of the plurality of rigid portions includes a circular cross-sectional profile.

5. The access device of claim 1 or claim 2, wherein each rigid portion of the plurality of rigid portions includes an elongate arcuate cross-sectional profile.

6. The access device of claim 5, wherein the cannula member defines a thickness, wherein each of the plurality of rigid portions extends the thickness of the cannula member.

7. The access device of any preceding claim, wherein the cannula member includes an inner layer.

8. The access device of claim 7, wherein the cannula member includes an outer layer.

9. The access device of claim 8, wherein the inner and outer layers form sleeve about the plurality of rigid and flexible portions.

10. The access device of any preceding claim, wherein the plurality of flexible portions are integrally formed.

11. The access device of any preceding claim, wherein the plurality of flexible portions form channels through which the plurality of rigid portions is received.
